(19) Europäisches Patentamt
European Patent Office
Office européen des brevets

(11) EP 0 852 952 A2

(12) # EUROPÄISCHE PATENTANMELDUNG

(43) Veröffentlichungstag:
**15.07.1998 Patentblatt 1998/29**

(51) Int. Cl.[6]: **A61L 31/00**, A61L 27/00, C08J 7/12

(21) Anmeldenummer: **97121167.7**

(22) Anmeldetag: **03.12.1997**

(84) Benannte Vertragsstaaten:
**AT BE CH DE DK ES FI FR GB GR IE IT LI LU MC NL PT SE**
Benannte Erstreckungsstaaten:
**AL LT LV MK RO SI**

(30) Priorität: **03.01.1997 DE 19700080**
**15.05.1997 DE 19720370**

(71) Anmelder:
**HÜLS AKTIENGESELLSCHAFT**
**45764 Marl (DE)**

(72) Erfinder:
- **Anders, Christine, Dr.**
  **45721 Haltern (DE)**
- **Lorenz, Günter, Dr.**
  **52068 Aachen (DE)**
- **Höcker, Hartwig, Prof. Dr.**
  **52076 Aachen (DE)**

(54) **Bioaktive Beschichtung von Oberflächen**

(57) Die Erfindung betrifft ein Verfahren zur bioaktiven Beschichtung der Oberfläche von Substraten, dadurch gekennzeichnet, daß man mindestens ein Monomer der allgemeinen Formel R-$(A)_a$, in der

R einen ein- oder zweifach olefinisch ungesättigten organischen Rest mit der Wertigkeit a bedeutet. A eine Carboxylgruppe -COOH, Schwefelsäuregruppe $-OSO_2OH$, Sulfonsäuregruppe $-SO_3H$, Phosphorsäuregruppe $-OPO(OH)_2$, Phosphonsäuregruppe $-PO(OH)_2$, Phosphorigsäuregruppe $-OP(OH)_2$, phenolische Hydroxylgruppe oder ein Salz oder einen Ester einer der genannten Gruppen bezeichnet und a für eine ganze Zahl von 1 bis 6 steht,

strahleninduziert auf einer aktivierten Substratoberfläche pfropfpolymerisiert, mit der Maßgabe, daß ein Monomer I, in dem A eine Carboxylgruppe -COOH oder ein Salz oder einen Ester der Carboxylgruppe bedeutet, entweder mindestens einen weiteren Rest A mit einer anderen der für A genannten Bedeutungen enthält oder zusammen mit mindestens einem weiteren Monomeren I verwendet wird, in dem A eine andere der für A genannten Bedeutungen hat. Das Verfahren eignet sich zur Herstellung von medizintechnischen oder biotechnischen Artikeln, Lagerbehältern oder Verpackungen.

EP 0 852 952 A2

## Beschreibung

Die Erfindung betrifft ein Verfahren zur bioaktiven Beschichtung der Oberfläche von Substraten, vorzugsweise von Kunststoff- (oder Polymer)substraten durch Pfropfpolymerisation. Eine wichtige Eigenschaft der erfindungsgemäß aufgebrachten Beschichtungen ist ihre gute Verträglichkeit im Kontakt mit Körperflüssigkeiten und Gewebe. Je nach Funktionalität der Beschichtungsmonomeren bzw. nach dem Molverhältnis bestimmter funktioneller Gruppen in der Beschichtung werden die Oberflächen darüber hinaus bakterienabweisend und zellproliferationshemmend oder bakterienabweisend und zellproliferationsfördernd. Die Erfindung betrifft weiterhin Gegenstände mit derart beschichteten Oberflächen sowie die Verwendung dieser Gegenstände für medizinische oder biotechnische Zwecke.

Die Ansiedelung und Vermehrung von Bakterien auf Oberflächen ist eine in der Regel unerwünschte Erscheinung, die häufig mit nachteiligen Folgen verbunden ist. So können in der Trinkwasser- und Getränketechnik Bakterienpopulationen zu einer gesundheitsgefährdenden Qualitätsminderung führen. Bakterien auf oder in Verpackungen bewirken häufig den Verderb von Lebensmitteln oder verursachen sogar Infektionen bei dem Verbraucher. In steril zu betreibenden biotechnischen Anlagen steilen systemfremde Bakterien ein erhebliches prozeßtechnisches Risiko dar. Solche Bakterien können mit Rohstoffen eingetragen werden oder bei mangelhafter Sterilisation in allen Anlageteilen zurückbleiben. Teile der Bakterienpopulation können sich durch Adhäsion dem normalen Flüssigkeitsaustausch beim Spülen und Reinigen entziehen und sich im System vermehren.

Weiterhin sind Bakterienansiedelungen in Wasseraufbereitungsanlagen (z.B. zur Entsalzung durch Membranen) oder auch in Behältern bekannt, die mit gelösten oder flüssigen unverdünnten organischen Substanzen gefüllt sind und für Bakterienpopulationen vorteilhafte Bedingungen aufweisen. Solche mikrobiellen Belegungen können in erheblichem Umfang zur Blockierung und/oder korrosiven Zerstörung der Anlage führen.

Besondere Bedeutung kommt dem Schutz vor Bakterienanhaftung und -ausbreitung in der Ernährung, der Pflege, hier insbesondere in der Altenpflege. und in der Medizin zu. Bei Massenbeköstigungen oder -ausschank existieren besonders dann erhebliche Risiken, wenn zur Vermeidung von Abfall von Einweggeschirr abgesehen wird und eine nur unzureichende Reinigung des Mehrweggeschirrs erfolgt. Die schädliche Ausbreitung von Bakterien in lebensmittelführenden Schläuchen und Rohren ist ebenso bekannt wie die Vermehrung in Lagerbehältern sowie in Textilien in feuchter und warmer Umgebung. z.B. in Bädern. Solche Einrichtungen sind bevorzugte Lebensräume für Bakterien, ebenso wie bestimmte Oberflächen in Bereichen mit hohem Publikumsverkehr, so z.B. in öffentlichen Verkehrsmitteln, Krankenhäusern, Telefonzellen. Schulen und insbesondere in öffentlichen Toiletten.

In der Alten- und Krankenpflege erfordern die häufig geminderten Abwehrkräfte der Betroffenen sorgfältige Maßnahmen gegen Infektionen, insbesondere auf Intensivstationen und in der häuslichen Pflege.

Besondere Sorgfalt bedarf die Verwendung medizinischer Gegenstände und Geräte bei medizinischen Untersuchungen. Behandlungen und Ein-griffen, vor allem dann, wenn derartige Geräte oder Gegenstände mit lebendem Gewebe oder mit Körperflüssigkeiten in Kontakt kommen. Im Falle von Langzeit- oder Dauerkontakten, beispielsweise bei Implantaten, Kathetern, Stents, Herzklappen und Herzschrittmachern, können Bakterienkontaminationen zu einem lebensbedrohenden Risiko für den Patienten werden.

Es wurde bereits auf vielfältige Weise versucht, die Ansiedelung und Ausbreitung von Bakterien auf Oberflächen zu unterbinden. In J. Microbiol. Chemoth. **31** (1993). 261-271 beschreiben S.E.Tebbs und T.S.J.Elliott lackartige Beschichtungen mit quaternären Ammoniumsalzen als antimikrobiell wirkenden Komponenten. Es ist bekannt, daß diese Salze von Wasser, wäßrigen oder anderen polaren Medien sowie von Körperflüssigkeiten aus dem Beschichtungsmaterial herausgelöst werden und ihre Wirkung somit nur von kurzer Dauer ist. Dies gilt gleichermaßen für die Einarbeitung von Silbersalzen in Beschichtungen, so beschrieben in WO 92/18098.

T. Ouchi und Y. Ohya beschreiben in Progr.Polym.Sci. **20** (1995), 211 ff., die Immobilisierung von bakteriziden Wirkstoffen auf Polymeroberflächen durch kovalente Bindung oder ionische Wechselwirkungen. Häufig sind in solchen Fällen die keimtötenden Wirkungen gegenüber dem reinen Wirkstoff deutlich reduziert. Heteropolare Bindungen erweisen sich oft als nicht hinreichend stabil. Darüber hinaus führt die Keimabtötung in der Regel zu unerwünschten Ablagerungen auf den Oberflächen, die die weitere bakterizide Wirkung maskieren und die Grundlage für eine nachfolgende Bakterienbesiedelung bilden.

W. Kohnen et al. berichten in ZBl. Bakt. Suppl. 26. Gustav Fischer Verlag, Stuttgart-Jena-New York, 1994, Seiten 408 bis 410, daß die Adhäsion von Staphylococcus epidermidis auf einem Polyurethanfilm vermindert wird, wenn der Film durch eine Glimmentladung in Gegen-wart von Sauerstoff vorbehandelt und dann mit Acrylsäure gepfropft wird.

Wie erwähnt, ist es wichtig, daß bei Verwendung medizinischer Gegenstände und Geräte bei medizinischen Untersuchungen, Behandlungen und Eingriffen eine Bakterienkontamination dieser Gegenstände und Geräte verhindert wird. Bei manchen dieser Gegenstände und Geräte. die mittel- oder langfristig mit lebendem Gewebe oder Körperflüssigkeiten in Kontakt kommen, ist zudem eine Haftung und Ausbreitung von körpereigenen Zellen ausgesprochen unerwünscht. So sind Zellbesiedelungen bei mittelfristig intrakorporal applizierten Kathetern ebenso schädlich wie bei langfristig implantierten Stents oder Herzklappen.

Weiterhin kann die Transparenz von Intraokularlinsen infolge von Zellbesiedelung nach der Implantation kontinuierlich abnehmen. Eine Reihe von Verfahren zielt darauf ab, eine Zellbesiedelung, beispielsweise durch die Einarbeitung bestimmter Metalle oder Metallsalze in die Halterung der Intraokularlinse zu vermeiden, wobei die Wirkung jedoch meist unvollständig und nicht nachhaltig ist. Auch in WO 94/16648 wird ein Verfahren beschrieben, durch das die Proliferation von Zellen auf der Oberfläche von implantierten Okularlinsen aus Polymermaterial verhindert werden soll.

Gemäß EP 0 431 213 sollen Polymere mit zellabweisenden Eigenschaften ausgestattet werden, indem ihre Oberfläche mit starken Mineralsäuren hydrophiliert wird. Die nachträgliche chemische Modifikation von Polymeroberflächen ist jedoch meist nicht gleichmäßig. Es bleiben in der Regel nicht oder nicht ausreichend behandelte Stellen zurück, die Ausgangspunkte für eine Zellbesiedelung bilden. Weiterhin sind die zellabweisenden Eigenschaften der so behandelten Oberflächen häufig nicht dauerhaft.

Andererseits sind für bestimmte Verwendungen Gegenstände mit Oberflächen erwünscht, die bakterienabweisend sind, aber die Besiedelung mit Zellen fördern. Das gilt z.B. für eine Reihe von Geräten für medizinische Untersuchungen, Behandlungen und Eingriffe und ebenso für manche Prothesen, die in das Gewebe einwachsen sollen, in das sie implantiert wurden. Solche Geräte und Prothesen. z.B. künstliche Hüftgelenke oder Zähne, bestehen häufig aus polymerummantelten Materialien, wie Titan.

Schließlich müssen Materialien für Geräte und Vorrichtungen, die mit Körperflüssigkeiten, wie Blut oder Lymphe, oder mit Gewebe in Kontakt kommen, verträglich für ihre fremde Umgebung sein. Insbesondere ist Blutverträglichkeit eine wichtige erwünschte Eigenschaft. Die Materialien müssen also möglichst ausgeprägte antithrombische Eigenschaften haben.

Es gibt also verschiedene, einander teilweise ausschließende Anforderungen an die bioaktiven Eigenschaften der Oberfläche von Polymeren, die für medizinische Verwendungen bestimmt sind. Sie sollen stets bakterienabweisend und verträglich mit Körperflüssigkeiten und Gewebe sein, aber wahlweise zellproliferationshemmend oder -fördernd wirken.

Der vorliegenden Erfindung lag die Aufgabe Zugrunde, ein verbessertes Verfahren zur Beschichtung von Oberflächen zu entwickeln, mit dem die Oberflächen bioaktiv, nämlich bakterienabweisend und verträglich für Körperflüssigkeiten und Gewebe sowie wahlweise zellproliferationshemmend oder -fördernd beschichtet werden können. ohne daß die mechanischen Eigenschaften der behandelten Materialien dadurch verändert werden oder sonstige erhebliche Nachteile eintreten.

Es wurde überraschenderweise gefunden, daß sich die Oberfläche von Substraten, insbesondere von polymeren Substraten, vorteilhaft bioaktiv beschichten läßt, wenn man mindestens ein Monomer der allgemeinen Formel

$$\text{Formel I:} \qquad R\text{-}(A)_a \quad ,$$

in der R einen ein- oder zweifach olefinisch ungesättigten organischen Rest, beispielsweise einen Kohlenwasserstoffrest, mit der Wertigkeit <u>a</u> bedeutet,

A eine Carboxylgruppe -COOH. Schwefelsäuregruppe $-OSO_2OH$, Sulfonsäuregruppe $-SO_3H$, Phosphorsäuregruppe $-OPO(OH)_2$, Phosphonsäuregruppe $-PO(OH)_2$, Phosphorigsäuregruppe $-OP(OH)_2$, phenolische Hydroxylgruppe oder ein Salz oder einen Ester einer der genannten Gruppen bezeichnet, und

<u>a</u> für eine ganze Zahl von 1 bis 6 steht.

strahleninduziert auf einer aktivierten Substratoberfläche pfropfpolymerisiert, mit der Maßgabe, daß ein Monomer I, in dem A die Carboxylgruppe -COOH oder ein Salz oder einen Ester der Carboxylgruppe bedeutet, entweder mindestens einen weiteren Rest A mit einer anderen der für A genannten Bedeutungen enthält oder zusammen mit mindestens einem weiteren Monomeren I verwendet wird, in dem A eine andere der für A genannten Bedeutungen hat.

Unter den Salzen der für A genannten Gruppen werden die Alkalisalze und insbesondere die Natriumsalze bevorzugt.

Das gemeinsame Kennzeichen der Monomeren der Formel I ist, daß sie 1 oder 2 olefinische Doppelbindungen sowie mindestens eine saure Gruppe oder ein bestimmtes Derivat, nämlich ein Salz oder einen Ester, einer sauren Gruppe aufweisen

Durch Plasma-induzierte Pfropfpolymerisation von funktionellen Monomeren erzeugte Beschichtungen auf verschiedenen Substraten sind z.B. aus B.Lassen et al., Clinical Materials **11** (1992), 99-103, bekannt und auf Biokompatibilitat untersucht worden. Eine aktivierende Vorbehandlung wird nicht erwähnt. Zudem ist Plasma kein optimaler Polymerisationsinitiator. H. Yasuda spricht dementsprechend in J.Polym.Sci.: Macromolecular Review. Vol. 16 (1981),

199-293, von der undefinierten und nicht kontrollierbaren Chemie der Plasma-Polymerisation. Dies mag für manche Zwecke akzeptabel sein, ist jedoch für medizinische und biotechnische Anwendungen problematisch, weil es gerade hier auf reproduzierbare Beschichtungen von gleichbleibend hoher Qualität besonders ankommt.

Überraschenderweise sind die bakterienabweisenden Eigenschaften der erfindungsgemäß mit einem carboxyl-, carboxylat- oder carbonestergruppenhaltigen Monomeren I zusammen mit einem anderen Monomeren I beschichteten Oberflächen deutlich stärker ausgeprägt als dies bei der Modifizierung mit Acrylsäure nach W. Kohnen et al., loc. cit., unter vergleichbaren Bedingungen der Fall ist.

Die erfindungsgemäß beschichteten Oberflächen zeigen eine bemerkenswerte Kombination vorteilhafter Eigenschaften und daher eine hervorragende physiologische Verträglichkeit. Sie sind insbesondere gut blutverträglich und vermindern die Adhäsion und Vermehrung von Bakterien in einem hohen Maße auch über längere Zeit. Von dieser Wirkung betroffene Bakterien sind u.a. Staphylococcus aureus. Staphylococcus epidermidis, Streptococcus pyogenes, Klebsiella pneumoniae, Pseudomonas aeroginosa und Escherichia coli. Gleichzeitig wird meistens auch die Proliferation von Zellen inhibiert, beispielsweise von Fibroblasten und Endothelzellen, wie menschlichen Nabelschnurzellen. Die besonderen Bedingungen, unter denen eine Beschichtung bakterienabweisend, aber zellproliferationsfördernd sind, werden später erläutert. Die Oberflächen der erfindungsgemäß beschichteten Substrate sind frei von migrationsfähigen und/oder extrahierbaren Monomer- und Oligomeranteilen. Unerwünschte Nebenwirkungen durch freigesetzte körperfremde Stoffe oder durch abgetötete Bakterien werden von vornherein vermieden.

Bei dem erfindungsgemäßen Verfahren werden die Substratoberflächen zunächst, wie in der Folge näher beschrieben, aktiviert und anschließend unter Einwirkung von UV-Licht durch schonende Pfropfpolymerisation oder -copolymerisation beschichtet.

## 1. Die Monomeren

Bei der Pfropf(co)polymerisation setzt man z.B. als Monomer I vorteilhaft eine Mischung von Monomeren der allgemeinen Formeln II oder III

$$\text{Formel II:} \quad (C_nH_{2n-q-x})(COOR^1)_x$$

$$\text{Formel III:} \quad (C_nH_{2n-q-x})(SO_3R^1)_x$$

ein. In den Formeln, die in der allgemeinen Formel I eingeschlossen sind, steht

<u>n</u> jeweils unabhängig für eine ganze Zahl von 2 bis einschließlich 6;

<u>x</u> jeweils unabhängig für 1 oder 2;

<u>q</u> jeweils unabhängig für 0 oder 2; und
bedeutet der Rest $R^1$ jeweils unabhängig -H, ein Äquivalent eines Metallions, insbesondere ein Alkalimetallion, einen Rest eines aliphatischen, cycloaliphatischen oder araliphatischen Alkohols, vorzugsweise eines Alkanols mit 1 bis 6, insbesondere mit 1 bis 4 Kohlenstoffatomen, eines Cycloalkanols mit 5 bis 12 Kohlenstoffatomen, eines Arylalkanols mit 7 bis 10 Kohlenstoffatomen oder eines Alkanols mit Sauerstoff- und/oder Stickstoffatomen in der Kette und bis zu 12 Kohlenstoffatomen, wie $-(CH_2-CH_2-O)_d-H$, $-(CH_2-CH(CH_3)-O)_d-H$, $-(CH_2-CH_2-CH_2-O)_d-H$ oder $-(CH_2)_d-NH_{2-e}(R^2)_e$, wobei $R^2$ für $-CH_3$ oder $-C_2H_5$, <u>d</u> für 1, 2, 3 oder 4 und <u>e</u> für 0, 1 oder 2 steht.

Den gegebenen Definitionen entsprechend bedeutet der Rest $(C_nH_{2n-q-x})$- jeweils unabhängig einen geradkettigen oder verzweigten einwertigen Alkenylrest (q=0, x=1) oder Alkadienylrest (q=2, x=1) oder einen zweiwertigen Alkenylenrest (q=0, x=2) oder Alkadienylenrest (q=2, x=2).

Anstelle von zwei Monomeren II und III kann man auch nur ein Monomer (II + III) einsetzen, das die Gruppen $-COOR^1$ und $-SO_3R^1$ in demselben Molekül enthält.

Auch vom Benzol abgeleitete Monomere der allgemeinen Formel

$$\text{Formel IV:} \quad (C_6H_{6-b-c-d})B_bR^3{}_c(OH)_d$$

können eingesetzt werden, worin

B jeweils unabhängig einen ein- oder zweiwertigen geradkettigen oder verzweigten Rest der Formeln $(C_nH_{2n-1-q-x})(COOR^1)_x$ oder $(C_nH_{2n-1-q-x})(SO_3R^1)_x$ bedeutet, wobei $R^1$, n, q und x wie zuvor definiert sind;

$R^3$ jeweils unabhängig $C_{1-4}$-Alkyl, $-NH_2$, -COOH, $-SO_3H$, $-OSO_3H$, $-OPO(OH)_2$, $-PO(OH)_2$, $-OP(OH)_2$, $-OPO(O^-)OCH_2-CH_2-N^+(CH_3)_3$, $-PO(O^-)O-CH_2-CH_2-N^+(CH_3)_3$, $-OP(O^-)OCH_2-CH_2-N^+(CH_3)_3$ oder gegebenenfalls ein Salz. insbesondere ein Alkalisalz, oder einen Ester der genannten Gruppen bedeutet;

b für 1, 2, oder 3 steht;

c für 0, 1, 2, oder 3 steht; und

d für 0, 1, 2, oder 3 steht;
mit der Maßgabe. daß $b + c + d \leq 6$, vorteilhaft $\leq 4$ ist.

Natürlich können auch beliebige Mischungen von Monomeren der allgemeinen Formeln II, III und IV für das erfindungsgemäße Verfahren eingesetzt werden.

Andere geeignete Monomere sind der Formel I entsprechende neutrale oder saure Schwefelsäureester und Salze der letzteren; Sulfonsäuren, deren Salze und Ester; Phosphonsäuren, deren neutrale oder saure Salze, neutrale oder saure Ester sowie Salze der letzteren; Phosphorsäureester, deren neutrale oder saure Salze, neutrale oder saure Ester sowie Salze der letzteren; und Phosphorigsäuren, deren neutrale oder saure Salze, neutrale oder saure Ester sowie Salze der letzteren. Auch diese Monomeren können im Gemisch untereinander und/oder mit den Monomeren der allgemeinen Formeln II, III und IV für das erfindungsgemäße Verfahren verwendet werden.

Schließlich seien noch 1 bis 3-wertige (oder basische) Phenole sowie deren Salze, die der Formel I entsprechen, als geeignete Monomere erwähnt. Auch sie werden gegebenenfalls im Gemisch untereinander und/oder mit den zuvor erwähnten Monomeren eingesetzt.

Für das erfindungsgemäße Verfahren hat sich eine Kombination aus Monomeren I bis IV bewährt, die zu Beschichtungen führt, welche einerseits Carboxyl- und/oder Carboxylatgruppen und andererseits Sulfonsäure- und/oder Sulfonatgruppen aufweisen. Es gibt unter dem Aspekt der Verträglichkeit hinsichtlich der genannten Gruppen drei mögliche Zweierkombinationen, nämlich Carboxyl- und Sulfonsäuregruppen, Carboxyl - und Sulfonatgruppen sowie Carboxylat- und Sulfonatgruppen, weiterhin zwei Dreierkombinationen, nämlich Carboxyl-, Carboxylat- und Sulfonatgruppen sowie Carboxyl-, Sulfosäure- und Sulfonatgruppen. Alle diese Kombinationen charakterisieren brauchbare Ausführungsformen des erfindungsgemäßen Verfahrens. Natürlich ist es auch möglich, Monomere einzusetzen, deren funktionelle Gruppen nach der Pfropfpolymerisation verändert werden. So kann man z.B. den Acrylamid-Baustein nachträglich durch Hydrolyse in saurem Medium in den Acrylsäure-Baustein umwandeln. Weiterhin kann man Carboxyl- und Sulfonsäuregruppen durch Neutralisieren (z.B. in Phosphatpuffern) sowie Carbonester- und Sulfosäureestergruppen durch Hydrolyse in Carboxylat- bzw. Sulfonatgruppen überführen.

In der genannten Kombination kann das molare Verhältnis von Carboxyl- und/oder Carboxylatgruppen zu Sulfonsäure- und/oder Sulfonatgruppen in der Beschichtung in weiten Grenzen schwanken. Besonders ausgeprägte zellproliferationsinhibierende Eigenschaften werden erzielt, wenn das genannte Verhältnis 0,2 bis 3, vorteilhaft 0,4 bis 3 und insbesondere 0,4 bis 2 beträgt. Die beschichteten Oberflächen zeigen in bemerkenswerter Weise bakterienabweisende, aber zellproliferationsfördernde Eigenschaften, wenn das besagte molare Verhältnis 2 bis 10, vorteilhaft 3 bis 10 und insbesondere 3 bis 5 beträgt. Zellproliferationsfördernd im Sinne der Erfindung ist eine Beschichtung dann, wenn die Haftung und Vermehrung von Säugetierzellen durch die Beschichtung im Vergleich zu der unbeschichteten Oberfläche verbessert oder jedenfalls weniger stark beeinträchtigt wird als die Haftung und Vermehrung von Bakterien.

Von den geeigneten Monomeren der allgemeinen Formeln I bis IV, die eine oder mehrere gleiche oder verschiedene Reste A im Molekül enthalten, seien beispielsweise genannt:
Acrylsäure, Natriumacrylat, Isobutylacrylat, 2-Ethylhexylacrylat, 2-Hydroxyethylacrylat, 2-(2'-Hydroxyethoxy)ethylacrylat, 2-Hydroxy-1-methylethylacrylat, 2-N,N-Dimethylaminoethylacrylat, Methacrylsäure, Natriummethacrylat, n-Propylmethacrylat, 2-Hydroxyethylmethacrylat, 2-(2'-Hydroxyethoxy)ethylmethacrylat, 2-Hydroxy-1-methylethylmethacrylat, 2-N,N-Dimethylaminoethylmethacrylat, Diethylenglykolmethacrylat, Triethylenglykoldiacrylat, 4-Vinylsalicylsäure, Itaconsäure, Vinylessigsäure, Zimtsäure, 4-Vinylbenzoesäure, 2-Vinylbenzoesäure, Sorbinsäure, Kaffeesäure, Maleinsäure, Methylmaleinsaure, Crotonsäure, Isocrotonsäure, Fumarsäure, Dimethylfumarsäure, Methylfumarsäure, Dihydroxymaleinsäure, Allylessigsäure;
Natriumallylsulfat, Natriumallylsulfonat, Natriummethallylsulfat Natriummethallylsulfonat, Natriumvinylsulfonat, Vinylsulfonsäure-2-hydroxyethylester, 4-Vinylbenzolsulfonsäure, Natriumstyrolsulfonat, Natriumvinyltoluolsulfonat;
Buten-(2)-diol-(1,4)-diphosphat, Buten-(2)-diol-(1,4)-diphosphonat, die Dinatriumsalze der beiden Phosphate bzw.

Phosphonate, Diallylphosphit;
2-Vinylphenol, 2-Allylhydrochinon, 4-Vinylresorcin, m-Hydroxystyrol, o-Hydroxystyrol, p-Hydroxystyrol, Carboxyl-vinylbenzolsulfonsäure.

Bei einer weiteren Ausführungsform des erfindungsgemäßen Verfahrens verwendet man als Monomer I eine Mischung aus Monomeren der allgemeinen Formeln V und VI

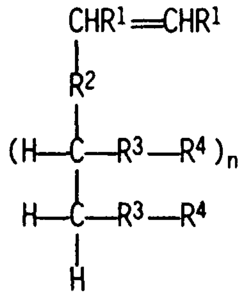 (V) und 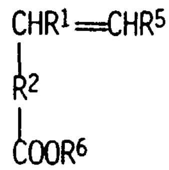 (VI)

In den Formeln I und II bedeuten

- $R^1$ Wasserstoff oder den Methylrest,
- $R^2$ einen zweiwertigen organischen Rest, vorzugsweise einen aliphatischen, cycloaliphatischen oder aromatischen Kohlenwasserstoffrest mit bis zu 10 Kohlenstoffatomen, oder eine C-C-Einfachbindung,
- $R^3$ -O- oder -NH-,
- $R^4$ Wasserstoff oder den Rest $-SO_3^-Na^+$,
- $R^5$ Wasserstoff, den Methylrest oder den Rest $-R^2-COO^-Na^+$,
- $R^6$ Wasserstoff oder Na und
- n 4 oder 5;

mit der Maßgabe, daß mindestens einer der Substituenten $R^4$ ein Rest $-SO_3^-Na^+$ ist.

In bevorzugten Monomeren V und VI bedeutet

- $R^1$ Wasserstoff,
- $R^2$ einen Alkylenrest mit 1 bis 4 Kohlenstoffatomen, einen Phenylenrest oder eine C-C-Einfachbindung,
- $R^3$ -O- oder -NH-,
- $R^4$ Wasserstoff oder den Rest $-SO_3^-Na^+$,
- $R^5$ Wasserstoff oder den Rest $-R^2-COO^-Na^+$,
- $R^6$ Wasserstoff oder Na und steht
- n für 4.

Die Monomeren V enthalten modifizierte Zuckerreste , vorzugsweise von Pentosen und insbesondere von Arabinose. Die Zuckerreste enthalten mindestens einen der Reste $-O-SO_3^-Na^+$ (O-Sulfat) oder $-NH-SO_3^-Na^+$ (N-Sulfat), bevorzugt benachbart zu dem Rest $R^2$. Sie weisen vorzugsweise 1 bis 4 dieser Reste auf. In einem Zuckerrest können O-Sulfat- und N-Sulfatreste gleichzeitig vorliegen, wobei dann der N-Sulfatrest bevorzugt benachbart zum Rest $R^2$ positioniert ist. Alternativ kann der Zuckerrest aber auch ausschließlich eine Art dieser Reste enthalten, z.B. nur O-Sulfatreste. Jede der genannten Spezies (nur O-Sulfat enthaltende sowie N-Sulfat-haltige Reste) ist für sich allein ode zusammen mit der anderen Spezies als Monomer V geeignet. Das Mischungsverhältnis beträgt also 0:100 bis 100:0.

Das Mengenverhältnis, in dem die Monomeren V und VI eingesetzt werden kann, in weiten Grenzen schwanken. So kann das Molverhältnis der N-Sulfat- und/oder O-Sulfatgruppen des Monomers V zu den Carboxyl- und/oder Carboxylatgruppen des Monomers VI bespielsweise 1:100 bis 100:1 betragen. Bevorzugte Molverhältnisse liegen zwischen 1:20 und 20:1.

Die Herstellung der Monomeren V ist in der deutschen Patentanmeldung **197 20 369.8** im einzelnen beschrieben. Sie sei hier anhand eines Spezialfalles erläutert, der von D-Glucono-1,5-lacton <u>1</u> ausgeht und zu einem Monomer V führt, das von einer Pentose, nämlich der D-Arabinose, abgeleitet ist. Der Fachmann wird jedoch das Verfahren ohne weiteres auf andere geeignete Edukte übertragen können.

In einer ersten Stufe werden die Hydroxylgruppen des Lactons <u>1</u> durch Acetalisierung geschützt, z.B. mit Aceton in Methanol als Lösemittel. Dabei wird das Lacton gespalten, und man erhält ein Isomerengemisch aus Methyl-3,4;5,6-di-O-isopropyliden-D-gluconat <u>2</u> und Methyl-2,3;5,6-di-O-isopropyliden-D-gluconat <u>3</u>. Dieses Gemisch wird in einer

**zweiten Stufe** reduziert, z.B. mit Lithiumaluminiumhydrid, wodurch die Carbonesterfunktion zur Carbinolfunktion wird. Man erhält wiederum ein Isomerengemisch, nämlich 3,4;5,6-Di-O-iso-propyliden-Dsorbit 4 und 2,3;5,6-Di-O-isopropyliden-D-sorbit 5. In einer **dritten Stufe** wird dieses Isomerengemisch mit einem Oxidationsmittel, wie Natriumperiodat, unter Spaltung der Kohlenstoffkette zu einem einheitlichen Produkt, dem Arabinosealdehyd 2,3;4,5-Di-O-isopropylidenaldehydo-D-arabinose 6 oxidiert. In der anschließenden **vierten Stufe** wird eine Vinylfunktion eingeführt, z.B. durch eine Grignard-Reaktion mit 4-Vinylphenylmagnesiumchlorid. Man erhält ein teilgeschütztes 4-Vinylphenylpentanpentaol, 2,3;4,5-Di-O-isopropyliden-1-(4-vinylphenyl)-D-gluco(D-manno)-pentitol **7**, das in der Folge kurz als Arasty bezeichnet wird.

Diese Stufenfolge 1 bis 4 wird durch das folgende Reaktionsschema veranschaulicht;

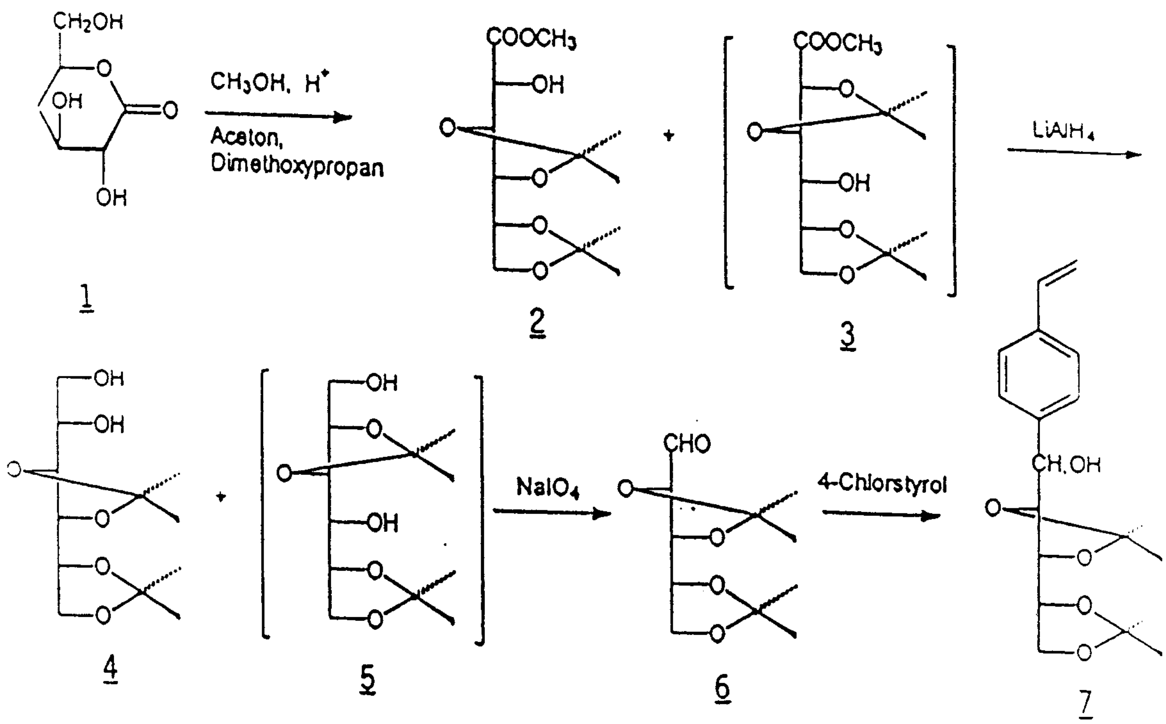

1 2 3 4 5 6 7

Diese Reaktionsfolge ist von H.Regeling et al., Recl.Trav.Chim. Pays-Bas **1987** (106), 461; D.Y.Jackson, Synth.Commun. **1988** (18), 337; und G.Wulff et al., Macromol.Chem.Phys. **1996** (197), 1285 näher beschrieben worden.

Zur Herstellung einer dem Arasty 7 entsprechenden Verbindung mit einer Aminogruppe in 1-Stellung kann man Arasty in einer **ersten Stufe** zum entsprechende Keton, (2,3;4,5-Di-O-isopropyliden-D-arabino)-(4vinylphenyl)-keton 8, oxidieren. Dieses wird in einer **zweiten** Stufe reduktiv zum Amin 1-Amino-1-desoxy-2,3;4,5-di-O-isopropyliden-1-(4vinylphenyl)-D-gluco(D-manno)-pentitol 9 umgewandelt. Diese Reaktionsfolge wird durch das folgende Formelschema erläutert:

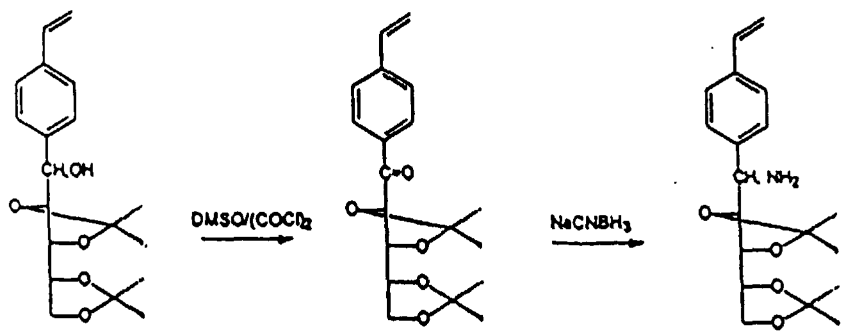


7 8 9

In der ersten Stufe kann Arasty 7 z.B. mit dem Komplex (?) aus Oxalylchlorid und Dimethylsulfoxid bei einer Temperatur von <-50°C in einem inerten Lösemittel oxidiert werden. Die reduktive Aminierung in der zweiten Stufe erreicht man vorteilhaft mit Natriumcyanoborhydrid als Reduktionsmittel in Gegenwart von Ammoniumacetat in einem Lösemittel unter Wasserausschluß bei Raumtemperatur.

Heparin enthält ungeschützte Hydroxylgruppen und ist O-sulfatiert und N-sulfatiert. Die Verbindungen 7 und 9 werden daher in einer **ersten Stufe** entschützt (deacetalisiert) und in einer **zweiten Stufe** Ound/oder N-sulfatiert, damit das aus ihnen hergestellte Polymer möglichst weitgehend heparinanalog ist. Die Entschützung gelingt in saurem Medium, in dem Ketale nicht beständig sind. Man erhitzt die geschützten Verbindungen z.B. mit verdünnter Mineralsäure oder einem sauren Ionenaustauscher und erhält aus 7 1-Hydroxy-1-desoxy-1-(4-vinylphenyl)-D-gluco(D-manno)-pentitol 10 und aus 9 das 1-Amino-1-desoxy-1-(4-vinylphenyl)-D-gluco(D-manno)-pentitol 11. Die Entschützung und die nachfolgende Sulfatierung werden durch das folgende Formelschema wiedergegeben:

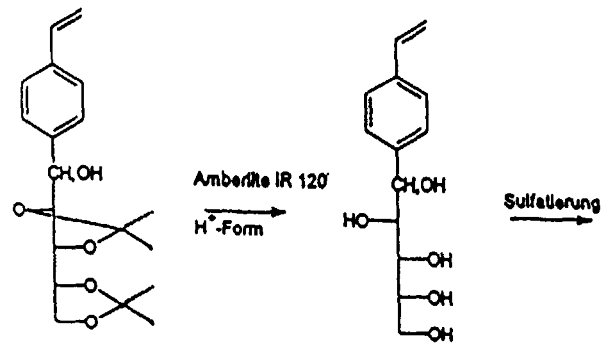


7 10 12

R=H oder $SO_3^-Na^+$

7

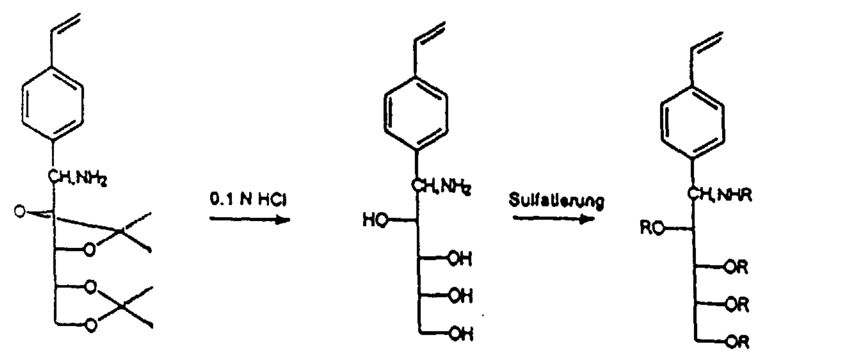


9 11 13

R=H oder $SO_3^-Na^+$

Die beiden Verbindungen <u>10</u> und <u>11</u> werden sulfatiert, zweckmäßig mittels eines Schwefeltrioxid-Pyridin-Komplexes. Wegen der vorweggenommenen Deacetalisierung kommt es bei der Sulfatierung nicht zu einem einheitlichen Produkt mit einer oder mehreren Sulfatgruppen in definierten Positionen. Es sollten jedoch die primären Hydroxylgruppen und die Aminogruppen bevorzugt sulfatiert werden. Durch Wahl eines geeigneten Molverhältnisses von Schwefeltrioxid zu Hydroxyl- bzw. Aminogruppen kann der Sulfatierungsgrad geregelt werden. Vorteilhaft wird durchschnittlich mehr als eine Sulfatgruppe pro Molekül eingeführt, da Heparin etwa 2,7 Sulfatgruppen pro Disaccharideinheit (entsprechend 1,35 Sulfatgruppen pro Molekül Monomer I) enthält. Durch Sulfatierung der entschützten Aminverbindung <u>11</u> erhält man gleichzeitig O-Sulfat- und N-Sulfatgruppen im Molekül, was im Hinblick auf die die angestrebte Heparinanalogie erwünscht ist.

Die Sulfatierung wird vorteilhaft bei Raumtemperatur durchgeführt, um eine vorzeitige Polymerisation zu vermeiden. Nach längerer Zeit, z.B. bis zu 100 Stunden, ist die Reaktion trotzdem vollständig. Als Lösemittel kann man z.B. überschüssiges Pyridin oder einen Ether, wie Tetrahydrofuran, verwenden. Da die Sulfatgruppen der Reaktionsprodukte säurelabil sind, empfiehlt es sich, der Eduktlösung vor dem Zusatz des Schwefeltrioxid-Pyridin-Komplexes ein wasserbindendes Mittel zuzusetzen, z.B. ein Molekularsieb. Aus demselben Grunde ist es empfehlenswert, nach Beendigung der Reaktion das Reaktionsgemisch zunächst durch Zusatz von Wasser und bald darauf einer Base (die den pH im alkalischen Bereich hält) zu hydrolysieren. Eine geeignete Base ist z.B. eine gesättigte Bariumhydroxidlösung, die zugleich Sulfationen ausfällt. Überschüssige Bariumionen können z.B. durch Einleiten von Kohlendioxid gefällt werden, gegebenenfalls nach vorsichtigem Einengen unter Entfernung von Lösemittel Das Bariumcarbonat wird abfiltriert und das Filtrat über eine Ionenaustauschersäule in der $Na^+$-Form gegeben oder anderweitig mit dem Ionenaustauscher behandelt, um die Bariumionen gegen Natriumionen auszutauschen. Aus der weiter eingeengten Lösung kann man durch Gefriertrocknen die Produkte, O-sulfatiertes 1-Hydroxy-1-desoxy-1-(4-vinylphenyl)-D-gluco(D-manno)-pentitol <u>12</u> bzw. N- und O-sulfatiertes 1-Amino-1-desoxy-1-(4vinylphenyl)-D-gluco(D-manno)-pentitol <u>13</u> jeweils in Form des

Natriumsalzes als pulverförmige Feststoffe gewinnen. Beide Stoffe entsprechen der Formel V und sind für die vorliegende Erfindung geeignete Monomere.

Bei den Monomeren VI handelt es sich um bekannte und gut zugängliche Stoffe, die die für die heparinanaloge Wirkung erforderlichen Carboxyl- oder Carboxylatgruppen beisteuern. Geeignete Monomere VI haben eine olefinische Doppelbindung und eine oder zwei Carboxyl- bzw. Carboxylatfunktionen oder Funktionen, die in Carboxyl- bzw. Carboxylatfunktionen umgewandelt werden können, wie Carbonester-, Carbonamid- oder Carbonsäureanhydridgruppen. Als Gegenionen zur Carboxylatfunktion dienen Natriumionen. Beispiele für geeignete Monomere II sind die Säuren (Meth)acrylsäure, Crotonsäure, 4-Vinylbenzoesäure, Maleinsäure, Fumarsäure, Itaconsäure, Vinylessigsäure, Zimtsäure, Crotonsäure, Isocrotonsäure, Methylmaleinsäure, Dimethylfumarsäure, Methylfumarsäure, Dihydroxymaleinsäure, Allylessigsäure sowie deren Natriumsalze.

2. **Weitere gegebenenfalls mitverwendbare Monomere**

Neben den beschriebenen Monomeren I bis V mit den aufgeführten blutverträglich machenden und bakterienabweisend bzw. zellproliferationsinhibierend wirkenden Gruppen kann man auch andere Monomere mitverwenden, die insoweit neutral oder allenfalls schwach wirksam sind. Dazu gehören z.B. Vinylether, wie Vinylmethylether und Vinylbutylether; Vinylketone, wie Vinylethylketon; Olefine und Diolefine, wie 1-Buten, 1-Hexen, 1,3-Butadien, Isopren und Chloropren; Acryl- und Methacrylamid; Vinylaromaten, wie Styrol, Vinyltoluol und Divinylbenzol; und Vinylsiloxane. Diese Monomeren können sogar in überwiegender Menge vorhanden sein, z.B. bis zu 90 mol% ausmachen.

3. **Die Substratmaterialien**

Als Substratmaterialien eignen sich vor allem alle polymeren Kunststoffe, wie Polyurethane, Polyamide, Polyester und -ether, Polyetherblockamide, Polystyrol, Polyvinylchlorid, Polycarbonate, Polyorganosiloxane, Polyolefine, Polysulfone, Polyisopren, Poly-Chloropren, Polytetrafluorethylen (PTFE), Polyacrylate, Polymethacrylate, entsprechende Copolymere und Blends sowie natürliche und synthetische Kautschuke, mit oder ohne strahlungssensitive Gruppen. Das erfindungsgemäße Verfahren läßt sich auch auf Oberflächen von lackierten oder anderweitig mit Kunststoff beschichteten Metall-, Glas- oder Holzkörpern anwenden.

3. **Die Aktivierung der Substratoberflächen**

Die Oberflächen der Substrate können erfindungsgemäß nach einer Reihe von Methoden aktiviert werden. Zweckmäßig werden sie zuvor in bekannter Weise mittels eines Lösemittels von Ölen. Fetten oder anderen Verunreinigungen befreit.

3.1 Die Aktivierung von Standardpolymeren ohne UV-strahlungssensitive Gruppen kann vorteilhaft durch UV-Strahlung, z.B. im Wellenlängenbereich von 100 bis 400 nm, vorzugsweise von 125 bis 310 nm erfolgen. Eine geeignete Strahlenquelle ist z.B. ein UV-Excimer-Gerät HERAEUS Noblelight, Hanau, Deutschland. Aber auch Quecksilberdampflampen eignen sich zur Substrataktivierung, sofern sie erhebliche Strahlungsanteile in den genannten Bereichen emittieren. Die Expositionszeit beträgt im allgemeinen 0,1 Sekunden bis 20 Minuten. vorzugsweise 1 Sekunde bis 10 Minuten. Es hat sich gezeigt, daß die Anwesenheit von Sauerstoff vorteilhaft ist. Die bevorzugten Sauerstoffdrücke liegen zwischen $2x10^{-5}$ und $2x10^{-2}$ bar. Man arbeitet beispielsweise in einem Vakuum von $10^{-4}$ bis $10^{-1}$ bar oder unter Verwendung eines Inertgases, wie Helium. Stickstoff oder Argon, mit einem Sauerstoffgehalt von 0,02 bis 20 Promille.

3.2 Die Aktivierung kann erfindungsgemäß auch durch ein Hochfrequenz- oder Mikrowellenplasma (Hexagon, Fa. Technics Plasma. 85551 Kirchheim, Deutschland) in Luft, Stickstoff- oder Argon-Atmosphäre erreicht werden. Die Expositionszeiten betragen im allgemeinen 30 Sekunden bis 30 Minuten, vorzugsweise 2 bis 10 Minuten. Der Energieeintrag liegt bei Laborgeräten zwischen 100 und 500W, vorzugsweise zwischen 200 und 300W.

3.3 Weiterhin lassen sich auch Korona-Geräte (Fa. SOFTAL, Hamburg, Deutschland) zur Aktivierung verwenden. Die Expositionszeiten betragen in diesem Falle in der Regel 1 Sekunde bis 10 Minuten. vorzugsweise 1 bis 60 Sekunden.

3.4 Die Aktivierung durch Elektronen- oder gamma-Strahlen (z.B. aus einer Kobalt-60-Quelle) ermöglicht kurze Expositionszeiten, die im allgemeinen 0,1 bis 60 Sekunden betragen.

3.5 Beflammungen von Oberflächen führen ebenfalls zu deren Aktivierung. Geeignete Geräte, insbesondere solche mit einer Barriere-Flammenfront, lassen sich auf einfache Weise bauen oder beispielsweise beziehen von der Fa. ARCOTEC, 71297 Mönsheim, Deutschland. Sie können mit Kohlenwasserstoffen oder Wasserstoff als Brenngas betrieben werden. In jedem Fall muB eine schädliche Überhitzung des Substrats vermieden werden, was durch innigen Kontakt mit einer gekühlten Metallfläche auf der von der Beflamnungsseite abgewandten Substratoberfläche leicht erreicht wird. Die Aktivierung durch Beflammung ist dementsprechend auf verhältnismäßig dünne, flächige Substrate beschränkt. Die Expositionszeiten belaufen sich im allgemeinen auf 0,1 Sekunde bis 1 Minute, vorzugsweise 0,5 bis 2 Sekunden, wobei es sich ausnahmslos um nicht leuchtende Flammen handelt und die Abstände der Substratoberflächen zur äußeren Flammenfront 0,2 bis 5 cm, vorzugsweise 0,5 bis 2 cm betragen.

3.6 Weiterhin lassen sich die Substratoberflächen auch durch Behandlung mit starken Säuren oder starken Basen aktivieren. Von den geeigneten starken Säuren seien Schwefelsäure, Salpetersäure und Salzsäure genannt. Man kann z.B. Polyamide 5 Sekunden bis 1 Minute mit konzentrierter Schwefelsäure bei Raumtemperatur behandeln. Als starke Basen eignen sich besonders Alkalimetallhydroxide in Wasser oder einem organischen Lösemittel. So kann man z.B. verdünnte Natronlauge 1 bis 60 Minuten bei 20 bis 80°C auf die Substrate einwirken lassen. Alternativ können beispielsweise Polyamide aktiviert werden, indem man 2 %iges KOH in Tetrahydrofuran 1 Minute bis 30 Minuten auf die Oberfläche einwirken läßt.

3.7 Schließlich können schon bei der Herstellung der Substratpolymeren Monomere mit UV-strahlungssensitiven Gruppen einpolymerisiert werden. Als solche eignen sich z.B. Furyl- oder Cinnamoylderivate, die z.B. in Mengen von 3 bis 10 mol% angewandt werden können. Gut geeignete Monomere dieser Art sind Cinnamoylethylacrylat und -methacrylat.

In manchen Fällen, z.B. bei hochhydrophoben Polymeren, kann es empfehlenswert sein, die Substratoberflächen durch eine Kombination aus zwei oder mehr der genannten Methoden zu aktivieren. Bevorzugte Aktivierungsmethoden sind die unter 3.1 und 3.2 genannten.

4. **Die Beschichtung durch Pfropf(co)polymerisation**

Wenn die Substrate nach einer der unter 3.1 bis 3.6 beschriebenen Methoden vorbehandelt wurden, werden die aktivierten Oberflächen zweckmäßig 1 bis 20 Minuten, vorzugsweise 1 bis 5 Minuten der Einwirkung von Sauerstoff, z.B. in Form von Luft, ausgesetzt.

Anschließend werden die (gegebenenfalls auch gemäß 3.7) aktivierten Oberflächen nach bekannten Methoden, wie Tauchen, Sprühen oder Streichen, mit Lösungen des oder der erfindungsgemäß zu verwendenden Vinylmonomeren beschichtet. Als Lösemittel haben sich Wasser und Wasser-Ethanol-Gemische bewährt, doch sind auch andere Lösemittel verwendbar, sofern sie ein ausreichendes Lösevermögen für die Monomeren aufweisen und die Substratoberflächen gut benetzen. Je nach Löslichkeit der Monomeren und gewünschter Schichtdicke der fertigen Beschichtung können die Konzentrationen der Monomeren in der Lösung 0,1 bis 50 Gewichtsprozent betragen. Lösungen mit Monomerengehalten von 3 bis 10 Gew.-%, beispielsweise mit etwa 5 Gew.-%, haben sich in der Praxis bewährt und ergeben im allgemeinen in einem Durchgang zusammenhängende, die Substratoberfläche bedeckende Beschichtungen mit Schichtdicken, die mehr als 0,1 μm betragen können.

Nach dem Verdampfen des Lösemittels oder auch schon während des Verdampfens wird die Polymerisation oder Copolymerisation der auf die aktivierten Oberflächen aufgebrachten Monomeren zweckmäßig durch Strahlen im kurzwelligen Segment des sichtbaren Bereiches oder im langwelligen Segment des UV-Bereiches der elektromagnetischen Strahlung bewirkt. Gut geeignet ist z.B. die Strahlung eines UV-Excimers der Wellenlängen 250 bis 500 nm, vorzugsweise von 290 bis 320 nm. Auch hier sind Quecksilberdampflampen geeignet, sofern sie erhebliche Strahlungsanteile in den genannten Bereichen emittieren. Die Expositionszeiten betragen im allgemeinen 10 Sekunden bis 30 Minuten, vorzugsweise 2 bis 15 Minuten.

Bisweilen ist es zweckmäßig die beschriebenen Arbeitsschritte einschließlich der Aktivierung zu wiederholen, um mittels einer solchen Mehrschichttechnik eine hermetisch geschlossene und/oder dickere Beschichtung sicherzustellen. Alternativ ist es auch möglich. das aktivierte Substrat, gegebenenfalls nach der beschriebenen Sauerstoffbehandlung, in die Lösung des oder der erfindungsgemäß zu verwendenden Vinylmonomeren einzutauchen und im getauchten Zustand zu bestrahlen. Durch orientierende Versuche läßt sich unschwer feststellen. bei welchen Bestrahlungszeiten mit einer gegebenen Strahlenquelle und bei welchen, gegebenenfalls längeren Kontaktzeiten von Substrat und Lösung die gewünschte Schichtdicke erreicht wird.

Das erfindungsgemäße Verfahren zur bakterienabweisenden und zellproliferationsinhibierenden Beschichtung der Oberfläche von Substraten und insbesondere von polymeren Kunststoffen gestattet die genaue Einstellung von Molverhältnissen verschiedener funktioneller Gruppen, die zur Inhibierung der Bakterienadhäsion und/oder -ausbreitung

sowie der Zellproliferation optimal sind. Darüber hinaus bietet das Verfahren den Vorteil, daß bei Wahl entsprechender Aktivierungsmethoden bereits bewährte Kunststoffe unter Beibehaltung ihrer mechanischen Eigenschaften und ihrer Form zusätzlich bakterienabweisend und zellproliferationsinhibierend modifizierbar sind. Im allgemeinen sind keine weiteren Vor- oder Nachbehandlungen erforderlich. Hochhydrophobe Kunststoffe bedürfen gegebenenfalls einer hydrophilierenden Vorbehandlung, z.B. durch chemisches Ätzen mit Säuren oder Basen oder durch Plasma-Behandlung, um eine ausreichende Benetzbarkeit durch die Monomerenlösung zu erreichen. Die hochhydrophoben Kunststoffe werden dann gleichzeitig hydrophiliert und im Sinne der vorliegenden Erfindung aktiviert.

Zur weiteren Erläuterung der vorliegenden Erfindung werden die folgenden Beispiele gegeben. Die darin verwendeten Monomeren sind stellvertretend für eine Vielzahl anderer unter die allgemeinen Formeln I bis IV fallenden Verbindungen.

## Beispiele

### Die verwendeten Monomeren

Von den in der Tabelle 1 aufgeführten Monomeren wurden jeweils 5 Gew-% wäßrige Lösungen unter sterilen Bedingungen hergestellt.

Tabelle 1

| Eingesetzte Monomere - 5 Gew.-% Lösung | | |
|---|---|---|
| Lösungsnummer | Monomer | Kurzzeichen |
| S 1 | Natriumstyrolsulfonat | NaSS |
| S 2 | Acrylsäure | AAc |
| S 3 | Methacrylsäure | MAAc |
| S 4 | Maleinsäure | MAc |
| S 5 | 2-[(N-Dimethylamino)ethyl]-acrylat | DMAEA |
| S 6 | Natriumvinylsulfonat | VS |
| S 7 | Methacryloyloxyethylphosphorylcholin, $CH_2=C(CH_3)-COO-CH_2CH_2OPO_2^{-}-CH_2-CH_2-N^{+}(CH_3)_3$ | PCHEMA |
| S 8 | Diethylenglykolmethacrylat | DEGMA |
| S 9 | Butadien-(1,3)-diol-(1,4)-diphosphat | BDD1,4DP |
| S 10 | Kaffeesäure | KafAc |
| S 11 * | 4-Vinylresorcin | 4VR |
| S 12 | 4-Vinylsalicylsäure | 4VSAC |

* 1 Gew.-% Lösung

### Die zu beschichtenden Substrate

Die Untersuchungen zu den Auswirkungen der erfindungsgemäßen Beschichtungen auf bakterienabweisendes Verhalten wurden an Folien aus den in Tabelle 2 aufgeführten Kunststoffen durchgeführt, die jeweils Stärken von 0,1 mm und eine für die Bestimmung relevante Oberfläche von 4 $cm^2$ besaßen. Sie wurden sowohl durch Lösen der Pulver in Lösemitteln, anschließendes Gießen in Petrischalen und Trocknen, als auch durch Kalandrieren, Extrudieren, Pressen oder Rakeln hergestellt. In einigen Fällen standen Folien vom Hersteller zur Verfügung.

Tabelle 2

| Eingesetzte Folien | | | |
|---|---|---|---|
| Folien-Nr. | Kunststoff | Name, Quelle | Herstellung |
| F 1 | Polyamid 12 | VESTAMID(R), HÜLS AG | Extrudieren |
| F 2 | Polystyrol | VESTYRON(R), HÜLS AG | Pressen |
| F 3 | Polyurethan | PELLETHANE(R) 2363-A DOW CHEMICAL COMPANY | Extrudieren |
| F 4 | Polyetherblockamid | VESTAMID(R), HÜLS AG | Extrudieren |
| F 5 | Polyethylen | VESTOLEN(R) A, VESTOLEN GmbH | Extrudieren |
| F 6 | Polypropylen | VESTOLEN(R) P, VESTOLEN GmbH | Extrudieren |
| F 7 | Polyorganosiloxan | NG 37-52, Silicon GmbH, Nünchritz | Rakeln |
| | | | |
| F 8 | Polyvinylchlorid | VESTOLIT(R) + DEHP, VESTOLIT GmbH | Brabandern |
| F 9 | PTFE | HOSTAFLON(R), HOECHST AG | Extrudieren |

**Die Aktivierung der Substratoberflächen**

Die Folien wurden zunächst aktiviert, wahlweise nach den Tabelle 3 angegebenen Verfahren und Bedingungen.

Tabelle 3

| Aktivierungsbedingungen | | |
|---|---|---|
| Aktivierungskennzeichen | Aktivierungsverfahren | Bedingungen |
| A 1 | UV-Excimer-Strahlen ( = 172 nm) | 1 s - 20 min, 1 mbar, 4 cm Abstand |
| A 2 | Mikrowellenplasma (Argon) | 1 s - 30 min, 1 mbar, |
| A 3 | Hochfrequenzplasma (Argon) | 1 s - 30 min, 6 mbar |
| A 4 | Corona | 0,1s - 60s, 2 mm Abstand |
| A 5 | Beflammen | $CH_4$:Luft=1:10;4cm Abstand |
| A 6 | μ-Strahlen | 1 MRad |
| A 7 | Elektronenstrahlen | 1 min |
| A 8 | NaOH-Lösung | 1%, 5 min, 60°C |
| A 9 | UV-Excimer-Strahlen ( = 308 nm) | 10 s - 20 min |

**Beschichtung der Substratoberflächen durch Pfropf(co)polymerisation**

Nach der Aktivierung werden die Folien, Gießkörper oder anderen Substratkörper sowie im Falle technischer Fertigung die Extrudate oder Spritzguß-Körper mit den Lösungen S1 bis S 16 beschichtet, und zwar nach den in Tabelle 4 angegebenen Verfahren.

Tabelle 4

| Beschichtungsverfahren | |
|---|---|
| Beschichtungskennzeichen | Beschichtungsverfahren |
| T 1 | Tauchen |

Tabelle 4 (fortgesetzt)

| Beschichtungsverfahren | |
|---|---|
| Beschichtungskennzeichen | Beschichtungsverfahren |
| T 2 | Sprühen |
| T 3 | Streichen |

Während des Tauchens oder nach dem Tauchen, Besprühen oder Bestreichen wird mit Strahlen im Bereich von 250 - 500 nm, vorzugsweise 290 - 320 nm bestrahlt.

**Bestimmung der bakterienabweisenden Eigenschaften**

Die Prüfung auf Adhäsion von Bakterien kann mit verschiedenen Stämmen vorgenommen werden. Hierzu eignen sich besonders die in Tabelle 5 aufgeführten Bakterien, da sie in klinischen Isolaten von infizierten Kathetern häufig vorkommen.

Tabelle 5

| Bakterienstämme zur Messung der primären Adhäsion | |
|---|---|
| | Stamm |
| B 1 | Staphylococcus aureus |
| B 2 | Staphylococcus epidermidis |
| B 3 | Escherichia coli |
| B 4 | Klebsiella pneumoniae |

Das Verfahren zur Bestimmung der primären Adhäsion (also unabhängig von späterer Vermehrung) dieser Bakterienstämme wird beispielhaft für Klebsiella pneumoniae in der Folge beschrieben. Die primäre Adhäsion der anderen Stämme (B1 bis B3) wurde analog bestimmt.

Bestimmung der primären Bakterienadhäsion unter statischen Bedingungen

Eine Über-Nacht-Kultur des Bakterienstammes Klebsiella pneumoniae in Hefeextrakt-Pepton-Glukose-Nährmedium (1% + 1% + 1%) wird abzentrifugiert und in Phosphat-gepufferter Saline (=PBS; 0,05m $KH_2PO_4$, pH 7,2 + 0,9 % NaCl) wieder aufgenommen. Man verdünnt mit PBS-Puffer auf ein Zellkonzentration von $10^8$ Zellen/ml. Die suspendierten Bakterien werden mit dem zu untersuchenden Folienstück für 3h in Berührung gebracht. Dazu werden doppelseitig beschichtete kreisförmige Folienstücke mit einem Durchmesser von 1,6 cm (=4,02 $cm^2$) auf eine Präpariernadel gesteckt und mit der Zellsuspension geschüttelt. Einseitig beschichtete Folien werden in Form einer runden, ebenen Scheibe von 4,5 cm Durchmesser und mit einer Stützmembran aus 2-3 cm dickem Weich-PVC in eine Membranfilterapparatur eingespannt. Auf die nach oben zeigende Seite mit der zu prüfenden Beschichtung wird die Zellsuspension aufgegeben und 3h geschüttelt. Die Membranfilterapparatur muß dicht sein, d.h. es darf keine Zellsuspension durch undichte Zellen ausfließen.

Nach Ablauf der Kontaktzeit wird die Bakteriensuspension mit einer Wasserstrahlpumpe abgesaugt, und die Folienstücke werden zum Waschen mit 20 ml steriler PBS-Lösung in einem 100 ml Becherglas 2 min geschüttelt. Das Folienstück wird nochmals in sterile PBS-Lösung eingetaucht und dann in 10 ml erhitztem TRIS/EDTA (0,1M Trishydroxyethylaminomethan, 4 mM Ethylendiamintetraessigsäure, mit HCl auf pH 7,8 eingestellt) für 2 min im siedenden Wasserbad extrahiert.

Mit der Extraktionslösung werden kleine Eppendorf-Cups befüllt und sofort bis zur Biolumineszenz-Bestimmung des extrahierten Adenosintriphosphats (ATP) bei -20°C eingefroren. Die Bestimmung wird wie folgt ausgeführt: In ein transparentes Röhrchen aus Polycarbonat wird 100 µl Reagentienmix (Biolumineszenz-Test CLS II, Fa. BOEHRINGER MANNHEIM GmbH) gegeben, und über einen Zeitraum von 10 sec werden in einem Lichtimpuls-Meßgerät LUMAT LB9501 (Laboratorien Prof. Berthold GmbH, 75323 Bad Wildbad, Deutschland) die Lichtimpulse integriert. Dann wird eine 100 µl Probe zugegeben und erneut gemessen. Die relativen Lichteinheiten (RLU) werden durch Subtraktion der Lichtimpulse im Reagentienmix von der Anzahl der gemessenen Lichtimpulse im kompletten Ansatz erhalten. Dieser

Wert steht in Relation zu der Anzahl der an der Folie adhärierten Bakterien. Der Umrechnungsfaktor zwischen dem RLU-Wert und der Bakterienzahl wird bestimmt, indem ein Aliquot von 0,1 ml der Bakteriensuspension mit $10^8$ Zellen/ml in 10 ml heißem TRIS/EDTA extrahiert und dann der ATP-Gehalt bestimmt wird.

Für Klebsiella pneumoniae ergibt sich eine Wert von $1{,}74 \cdot 10^4$ RLU = $1 \cdot 10^7$ Zellen im ATP-Extraktionsansatz. Bei einem gemessenen Wert von $4{,}7 \cdot 10^4$ RLU von 4 $cm^3$ Folie waren pro $cm^2$ Folienoberfläche

$$\frac{4{,}7 \cdot 10^4}{4} = 1{,}175 \cdot 10^4 \text{ RLU/cm}^2 = 6{,}8 \cdot 10^6 \text{ Zellen/cm}^2$$

primär adhäriert.

**Ergebnisse**

In den folgenden Tabellen 6a und 6b sind die verschiedenen Bedingungen und die Ergebnisse von insgesamt 27 Versuchen und Vergleichsversuchen ohne vorherige Aktivierung (14, 16, 18, 20, 22, 24, 26) zusammengestellt.

| Versuch | Substrat | Aktivierung | Monomer [Vol/Vol. 5 Gew.-% Lösung] | Beschichtung | Bakterienadhäsion (10 000 Zellen/cm²) | | | | | | | |
|---|---|---|---|---|---|---|---|---|---|---|---|---|
| | | | | | B 1 | | B 2 | | B 3 | | B 4 | |
| | | | | | Wert | Δ [%] | Wert | Δ [%] | Wert | Δ [%] | Wert | Δ [%] |
| 14 | 3 F | - | - | - | 270 | - | 210 | - | 340 | - | 470 | - |
| 15 | 3 F | A 1, 5 min | S 1:S 2= 9 : 1 | T 1 | 2,9 | 99 | 2,8 | 99 | 4,3 | 99 | 5,8 | 99 |
| 16 | 4 F | - | - | - | 180 | - | 360 | - | 560 | - | 300 | - |
| 17 | 4 F | A 8, 5 min | S 1:S 4 = 1:1 | T 2 | 36 | 80 | 65 | 82 | 78 | 86 | 51 | 83 |
| 18 | 5 F | - | - | - | 230 | - | 290 | - | 680 | - | 710 | - |
| 19 | 5 F | A 1, 5 min. | S 1 : S 8 = 10 : 1 | T 2 | 10 | 96 | 5,8 | 98 | 33 | 95 | 50 | 93 |
| 20 | 6 F | - | - | - | 260 | - | 300 | - | 730 | - | 780 | - |
| 21 | 6 F | A 3, 7 min | S 6:S10 = 1:1 | T 3 | 5,4 | 98 | 4,2 | 99 | 54 | 93 | 10 | 99 |
| 22 | 7 F | - | - | - | 1400 | - | 1200 | - | 1900 | - | 3000 | - |
| 23 | 7 F | A 6 | S5 : S 12 = | T 1 | 670 | 52 | 490 | 59 | 850 | 53 | 1350 | 55 |
| 24 | 8 F | - | - | - | 130 | - | 110 | - | 200 | - | 310 | - |
| 25 | 8 F | A 5, 2 sec | S 7 | T 3 | 20 | 85 | 15 | 86 | 32 | 84 | 52 | 83 |
| 26 | 9 F | - | - | - | 1100 | - | 970 | - | 1700 | - | 3400 | - |
| 27 | 9 F | A 7, 1 min | S 3:S 6 = 10:3 | T 1 | 520 | 53 | 190 | 80 | 760 | 55 | 1660 | 51 |

Tab. 6b : Aktivierungs-, Beschichtungsverfahren und Bakterienmessungen an verschiedenen Polymeren
Δ [%] = Differenz zum unbehandelten Substrat in [%]

| Versuch | Aktivierung Art, Dauer | Bakterienadhäsion [10.000 Zellen/cm²] | | | | | | | |
|---|---|---|---|---|---|---|---|---|---|
| | | B 1 | | B 2 | | B 3 | | B 4 | |
| | | Wert | Δ [%] | Wert | Δ [%] | Wert | Δ [%] | Wert | Δ [%] |
| 1 | - | 310 | | 500 | | 600 | | 720 | |
| 2 | A 1, 1 min | 36 | 88 | 93 | 81 | 100 | 83 | 140 | 81 |
| 3 | A 1, 3 min | 24 | 92 | 47 | 91 | 59 | 90 | 63 | 91 |
| 4 | A 1, 5 min | 7,1 | 98 | 13 | 97 | 12 | 98 | 14 | 98 |
| 5 | A 2, 2 min | 44 | 86 | 120 | 76 | 370 | 38 | 420 | 42 |
| 6 | A 2, 4 min | 28 | 91 | 97 | 81 | 99 | 83 | 120 | 83 |
| 7 | A 2, 6 min | 12 | 96 | 36 | 93 | 56 | 91 | 64 | 91 |
| 8 | A 4, 2 m/min | 58 | 81 | 74 | 85 | 81 | 86 | 85 | 88 |
| 9 | A 4, 2 m/min | 55 | 82 | 68 | 86 | 75 | 87 | 67 | 91 |
| 10 | A 4, 1 m/min | 49 | 84 | 60 | 88 | 63 | 89 | 65 | 91 |
| 11 | A 9, 1 min | 63 | 80 | 110 | 78 | 180 | 70 | 200 | 72 |
| 12 | A 9, 3 min | 53 | 83 | 93 | 81 | 97 | 84 | 110 | 85 |
| 13 | A 9, 5 min | 41 | 87 | 78 | 84 | 81 | 86 | 82 | 89 |

Tab. 6a: Beschichtungen von Polyamid 12 (1 F) durch Tränken (T 1) mit Natriumstyrolsulfonat/Acrylsäure (S 1:S 2 = 1:9 [Vol/Vol]) unter verschiedenen Aktivierungsbedingungen und die Ergebnisse der Bakterienadhäsionen (B 1 - B 4)

Δ [%] = Differenz zum unbehandelten Polyamid 12 (1 F) in %
= Reduzierung der Bakterienadhäsion in %

Die Tabellen 6a und 6b verdeutlichen, daß nach dem erfindungsgemäßen Verfahren Beschichtungen erhalten werden, die zu einer erheblichen Reduzierung der Bakterienadhäsion führen. Die Minderungen liegen deutlich über 50%

im Vergleich zu den unbeschichteten Substraten. Weiterhin lassen die Vergleichsbeispiele (Versuche 3, 4 und 15) erkennen, daß durch Aktivierung der Substratoberflächen mit UV-Excimer-Strahlen der Wellenlänge 172 nm (A1) oder Plasmabehandlung (A2, A3) überraschenderweise höhere Inhibierungen der Bakterienadhäsion (≥90%) erreicht werden als mit anderen Aktivierungsmitteln. wie Elektronenstrahlen (A7, Versuch 13) oder NaOH-Lösungen (A8, Versuch 17).

Die in den Tabellen wiedergegebenen Ergebnisse zeigen weiter, daß man mit einem einzigen Monomer erfolgreich arbeiten kann (S7, Versuch 25), daß aber auch Copolymerisate aus zwei Monomeren, nämlich aus Natriumstyrolsulfonat und Acrylsäure (S 1 + S 2; Versuch 15), Natriumstyrolsulfonat und Maleinsäure (S 1 + S 4; Versuch 17), Natriumvinylsulfonat und Kaffeesäure (S 6 + S 10; Versuch 21) sowie Acrylsäure und Natriumvinylsulfonat (S 2 + S 6; Versuch 27) Beschichtungen mit guten bakterienabweisenden Eigenschaften ergeben.

**Patentansprüche**

1. Verfahren zur bioaktiven Beschichtung der Oberfläche von Substraten, dadurch gekennzeichnet, daß man mindestens ein Monomer der allgemeinen Formel

$$\text{Formel I:} \qquad R\text{-}(A)_a \ .$$

in der R einen ein- oder zweifach olefinisch ungesättigten organischen Rest mit der Wertigkeit a bedeutet.

A eine Carboxylgruppe -COOH, Schwefelsäuregruppe $-OSO_2OH$, Sulfonsäuregruppe $-SO_3H$, Phosphorsäuregruppe $-OPO(OH)_2$, Phosphonsäuregruppe $-PO(OH)_2$, Phosphorigsäuregruppe $-OP(OH)_2$, phenolische Hydroxylgruppe oder ein Salz oder einen Ester einer der genannten Gruppen bezeichnet und

a für eine ganze Zahl von 1 bis 6 steht,

strahleninduziert auf einer aktivierten Substratoberfläche pfropfpolymerisiert, mit der Maßgabe. daB ein Monomer I, in dem A eine Carboxylgruppe -COOH oder ein Salz oder einen Ester der Carboxylgruppe bedeutet, entweder mindestens einen weiteren Rest A mit einer anderen der für A genannten Bedeutungen enthält oder zusammen mit mindestens einem weiteren Monomeren I verwendet wird, in dem A eine andere der für A genannten Bedeutungen hat.

2. Verfahren nach Anspruch 1, dadurch gekennzeichnet, daß man man als Monomer I eine Mischung von Monomeren der allgemeinen Formeln II oder III

$$\text{Formel II :} \qquad (C_nH_{2n-q-x})(COOR^1)_x$$
$$\text{Formel III:} \qquad (C_nH_{2n-q-x})(SO_3R^1)_x$$

verwendet, wobei

- n jeweils unabhängig für eine ganze Zahl von 2 bis einschließlich 6;
- x jeweils unabhängig für 1 oder 2;
- q jeweils unabhängig für O oder 2 steht und der Rest $R^1$ jeweils unabhängig -H, ein Äquivalent eines Metallions oder einen Rest eines aliphatischen, cycloaliphatischen oder araliphatischen Alkohols bedeutet.

3. Verfahren nach Anspruch 1, dadurch gekennzeichnet, daß man man als Monomer I ein von Benzol abgeleitetes Monomer der allgemeinen Formel

Formel IV: $(C_6H_{6-b-c-d})B_bR^3_c(OH)_d$

verwendet, in der

B jeweils unabhängig einen ein- oder zweiwertigen geradkettigen oder verzweigten Rest der Formeln $(C_nH_{2n-1-q-x})(COOR^1)_x$ oder $(C_nH_{2n-1-q-x})(SO_3R^1)_x$ bedeutet, wobei $R^1$, n, q und x wie in Anspruch 2 definiert sind;

$R^3$ jeweils unabhängig $C_{1-4}$-Alkyl, $-NH_2$, -COOH, $-SO_3H$, $-OSO_3H$, $-OPO(OH)_2$, $-PO(OH)_2$, $-OP(OH)_2$, $-OPO(O^-)OCH_2-CH_2-N^+(CH_3)_3$, $-PO(O^-)O-CH_2-CH_2-N^+(CH_3)_3$, $-OP(O^-)OCH_2-CH_2-N^+(CH_3)_3$ oder gegebenenfalls ein Salz oder einen Ester der genannten Gruppen bedeutet;

b für 1, 2, oder 3 steht;

c für 0, 1, 2, oder 3 steht; und

d für 0, 1, 2, oder 3 steht;

mit der Maßgabe, daß $b + c + d \leq 6$ ist.

4. Verfahren nach Anspruch 1, dadurch gekennzeichnet, daß man als Monomer I eine Mischung aus Monomeren der allgemeinen Formeln V und VI

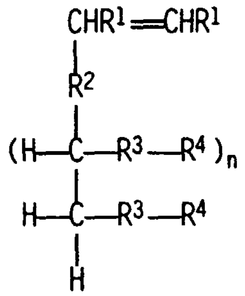 (V) und 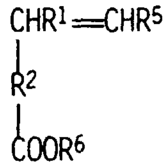 (VI)

verwendet, in denen

$R^1$ Wasserstoff oder den Methylrest,

$R^2$ einen zweiwertigen organischen Rest oder eine C-C-Einfachbindung,

$R^3$ -O- oder -NH-,

$R^4$ Wasserstoff oder den Rest $-SO_3^-Na^+$

$R^5$ Wasserstoff, den Methylrest oder de$^n$ Rest $-R^2-COOR^6$,

$R^6$ Wasserstoff oder Na und

n 4 oder 5 bedeuten;

mit der Maßgabe, daß mindestens einer der Substituenten $R^4$ ein Rest $-SO_3^-Na^+$ ist.

5. Verfahren nach Anspruch 2, dadurch gekennzeichnet, daß die Monomeren II und III so gewählt werden, daß die gepfropfte Polyinerschicht (iii) Carboxyl- und Sulfonsäuregruppen, (iv) Carboxyi- und Sulfonatgruppen, (v) Carboxylat- und Sulfonatgruppen, (vi) Carboxyl-, Carboxylat- und Sulfonatgruppen oder Carboxyl-, Sulfosäure- und Sulfonatgruppen enthält.

6. Verfahren nach Anspruch 5, dadurch gekennzeichnet, daß das molare Verhältnis von Carboxyl- und/oder Carboxylatgruppen zu Sulfonsäure und/oder Sulfonatgruppen 0,2 bis 3 beträgt.

7. Verfahren nach Anspruch 5, dadurch gekennzeichnet, daß das molare Verhältnis von Carboxyl- und/oder Carboxylatgruppen zu Sulfonsäure und/oder Sulfonatgruppen 0,4 bis 3 beträgt.

8. Verfahren nach Anspruch 5, dadurch gekennzeichnet, daß das molare Verhältnis von Carboxyl- und/oder Carboxylatgruppen zu Sulfonsäure und/oder Sulfonatgruppen 0,4 bis 2 beträgt.

9. Verfahren nach Anspruch 5, dadurch gekennzeichnet, daß das molare Verhältnis von Carboxyl- und/oder Carboxylatgruppen zu Sulfonsäure und/oder Sulfonatgruppen 2 bis 10 betragt.

10. Verfahren nach Anspruch 5, dadurch gekennzeichnet, daß das molare Verhältnis von Carboxyl- und/oder Carboxylatgruppen zu Sulfonsäure und/oder Sulfonatgruppen 3 bis 10 beträgt.

11. Verfahren nach Anspruch 5, dadurch gekennzeichnet, daß das molare Verhältnis von Carboxyl- und/oder Carboxylatgruppen zu Sulfonsäure und/oder Sulfonatgruppen 3 bis 5 beträgt.

12. Verfahren nach Anspruch 1, dadurch gekennzeichnet, daß das Monomer I bzw. die Monomeren I so gewählt werden, daß die gepfropfte Polymerschicht Phosphorsäuregruppen oder Phosphonsäuregruppen oder deren Salze oder Ester enthält.

13. Verfahren nach einem der Ansprüche 1 bis 12, dadurch gekennzeichnet, daß die Aktivierung der Substratoberflächen durch in das Substratpolymer einpolymerisierte Monomere mit einer UV-strahlungssensitiven Gruppe, durch UV-Strahlung, Plasmabehandlung, Coronabehandlung, Elektronenstrahlbehandlung, Beflammen und bzw. oder Behandlung mit einer starken Säure oder starken Base erfolgt.

14. Verfahren nach einem der Ansprüche 1 bis 12, dadurch gekennzeichnet, daß die Aktivierung der Substratoberfläche durch UV-Strahlung im Wellenlängenbereich von 100 bis 400 nm bei einer Expositionszeit von 0,1 Sekunden bis 20 Minuten erfolgt.

15. Verfahren nach einem der Ansprüche 1 bis 12, dadurch gekennzeichnet, daß die Aktivierung der Substratoberfläche durch UV-Strahlung im Wellenlängenbereich von 125 bis 310 nm bei einer Expositionszeit von von 1 Sekunde bis 10 Minuten erfolgt.

16. Verfahren nach einem der Ansprüche 1 bis 12, dadurch gekennzeichnet, daß die Aktivierung der Substratoberfläche durch Hochfrequenz- oder Mikrowellenplasma bei einer Expositionszeit von 30 Sekunden bis 30 Minuten erfolgt.

17. Verfahren nach einem der Ansprüche 1 bis 14, dadurch gekennzeichnet, daß man die aktivierten Oberflächen vor der Beschichtung 1 bis 20 Minuten der Einwirkung von Sauerstoff aussetzt.

18. Verfahren nach Anspruch 17, dadurch gekennzeichnet, daß man den Sauerstoff 1 bis 5 Minuten einwirken läßt.

19. Verfahren nach einem der Ansprüche 1 bis 18, dadurch gekennzeichnet, daß man die Polymerisation der Monomeren durch Strahlen im Bereich von 250 bis 500 nm bewirkt.

20. Verfahren nach einem der Ansprüche 1 bis 18, dadurch gekennzeichnet, daß man die Polymerisation der Monomeren durch UV-Strahlen im Bereich von 290 bis 320 nm bewirkt.

21. Verwendung von Substraten mit gemäß den Ansprüchen 1 bis 20 beschichteten Oberflächen gemäß den Ansprüche 1 bis 18 zur Herstellung von medizintechnischen oder biotechnischen Artikeln, Lagerbehältern oder Verpakkungen.

22. Verwendung nach Anspruch 21, dadurch gekennzeichnet, daß die Artikel Katheter, Schläuche oder Rohrleitungen sind.